(19) Europäisches Patentamt
European Patent Office
Office européen des brevets

(11) Veröffentlichungsnummer : **0 113 911**
**B1**

(12) # EUROPÄISCHE PATENTSCHRIFT

(45) Veröffentlichungstag der Patentschrift : **26.03.86**

(21) Anmeldenummer : **83112974.7**

(22) Anmeldetag : **22.12.83**

(51) Int. Cl.[4] : **C 07 D471/14**, A 61 K 31/505 // (C07D471/14, 249:00, 239:00, 221:00)

(54) **Pyrido-triazolochinazoline, ihre Herstellung und Verwendung.**

(30) Priorität : **08.01.83 DE 3300477**

(43) Veröffentlichungstag der Anmeldung : **25.07.84 Patentblatt 84/30**

(45) Bekanntmachung des Hinweises auf die Patenterteilung : **26.03.86 Patentblatt 86/13**

(84) Benannte Vertragsstaaten : **AT BE CH DE FR IT LI LU NL SE**

(56) Entgegenhaltungen :
**DE-A- 2 757 929**
**GB-A- 1 512 299**

(73) Patentinhaber : **BOEHRINGER INGELHEIM KG**
**D-6507 Ingelheim am Rhein (DE)**

(72) Erfinder : **Schromm, Kurt, Dr.**
**In der Dörrwiese 35**
**D-6507 Ingelheim (DE)**
Erfinder : **Mentrup, Anton, Dr.**
**Steinerstrasse 25**
**D-6503 Mainz-Kastel (DE)**
Erfinder : **Renth, Ernst-Otto, Dr.**
**Frankenstrasse 11**
**D-6507 Ingelheim (DE)**
Erfinder : **Fügner, Armin, Dr.**
**Im Hippel 31**
**D-6535 Gau-Algesheim (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue heterocyclische Verbindungen der Formel

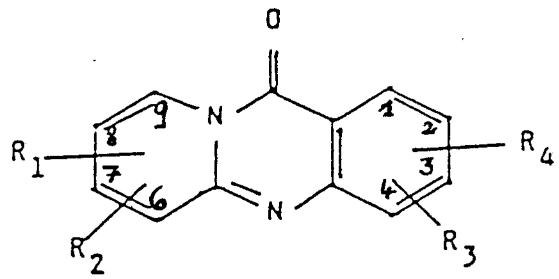

(I)

in der entweder $R_1$ und $R_2$ für Wasserstoff, niederes Alkyl, niederes Alkoxy, $R_1$ auch für Halogen in 8-Position (nach der Numerierung in Formel I), $R_2$ auch für einen ankondensierten Benzolring und $R_3$ und $R_4$ gemeinsam für die Gruppe —N=N—NH— stehen oder $R_1$ und $R_2$ gemeinsam die Gruppe —N=N—NH— bedeuten und $R_3$ und $R_4$ Wasserstoff, niederes Alkyl, niederes Alkoxy, $R_3$ auch Halogen in 2-, 3- oder 4-Position (nach der Numerierung in Formel I), $R_4$ auch einen ankondensierten Benzolring darstellen, sowie die Salze dieser Verbindungen mit basischen Stoffen.

Die Gruppe —N=N—NH— ist jeweils mit zwei benachbarten C-Atomen verknüpft, so daß folgende Grundstrukturen vorliegen können :

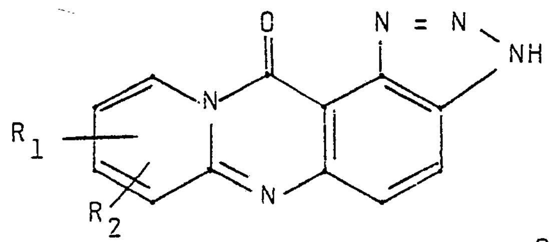

(Ia)

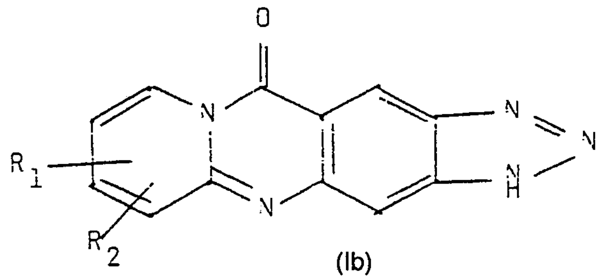

(Ib)

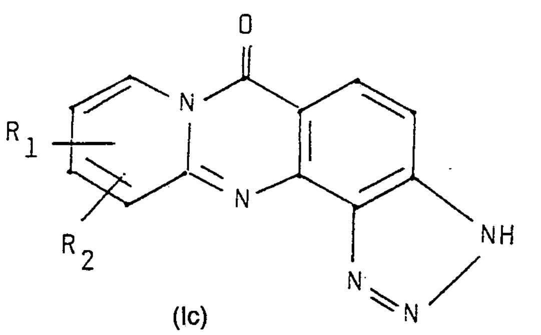

(Ic)

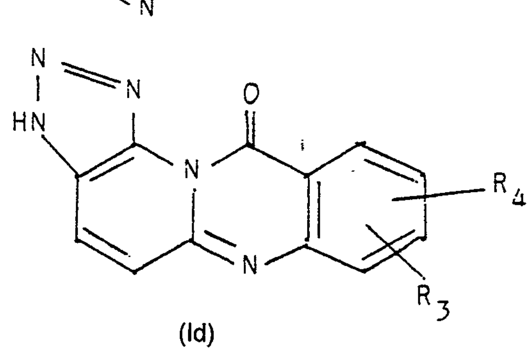

(Id)

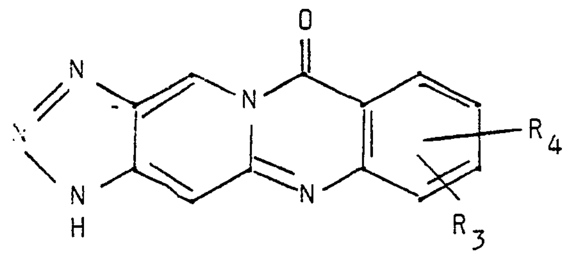

(Ie)

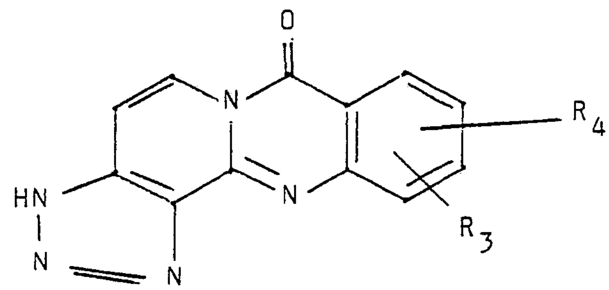

(If)

Entsprechend kann, falls $R_1$ bzw. $R_3$ einen ankondensierten Benzolring darstellen, dieser jeweils an zwei bzw. drei Seiten ankondensiert sein, d. h. in 6/7- oder 8/9- bzw. in 1/2-, 2/3- oder 3/4-Position der Formel I.

Die Reste $R_1$ und $R_2$ bzw. $R_3$ und $R_4$ können, soweit sie für Wasserstoff, Halogen, niedere Alkyl- oder niedere Alkoxyreste stehen, gleich oder verschieden sein.

Die neuen Verbindungen werden nach an sich bekannten Verfahren erhalten.

1. Man setzt eine Diaminoverbindung der Formel

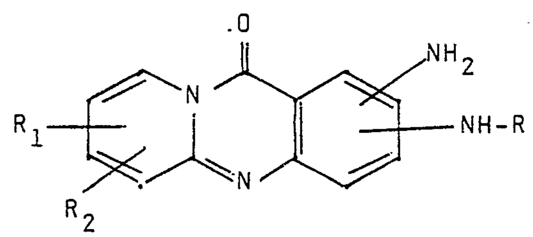

(IIa)

bzw.

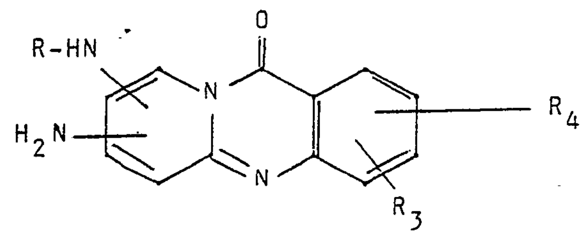

(IIb)

(R = H oder Acyl, insbesondere Acetyl und andere aliphat. Acyle mit bis zu 6 C-Atomen)

worin $R_1$ und $R_2$ bzw. $R_3$ und $R_4$ die obige Bedeutung haben und die Aminogruppen sich jeweils an benachbarten C-Atomen befinden, in Lösung, z. B. in Eisessig oder anderen geeigneten Lösungsmitteln, mit nitrosierenden Agenzien, beispielsweise wäßriger Natriumnitritlösung oder auch N-Nitrosodiphenylamin, bei Temperaturen zwischen etwa 0 und 100 °C in Gegenwart von Säuren um. Wird Eisessig als Lösungsmittel benutzt, so übernimmt dieser zugleich die Funktion der Säure. Verwendet man andere Lösungsmittel, die selbst keine Säuren sind, wird z. B. Eisessig oder Salzsäure zugegeben.

2. Man cyclisiert eine Verbindung der Formel

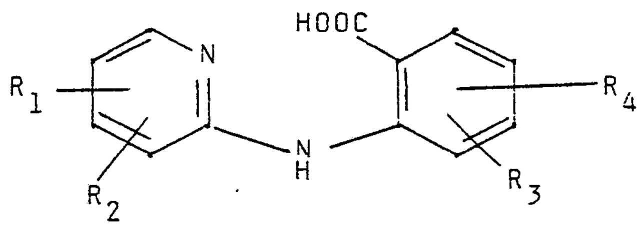

(III)

worin $R_1$ bis $R_4$ die obige Bedeutung haben, durch Einwirkung von Säuren, insbesondere Mineralsäuren, etwa Salzsäure.

Es ist jedoch nicht erforderlich, die Verbindungen der Formel III unmittelbar einzusetzen. Vielmehr ist es im allgemeinen zweckmäßiger, von Vorstufen auszugehen, aus denen in situ die Verbindungen der Formel III entstehen (die dann nicht zwischenisoliert werden müssen).

Man kann somit Anthranilsäuren der Formel

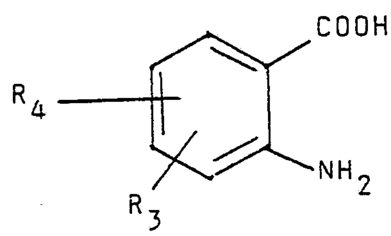

(IV)

und 2-Halogenpyridine der Formel

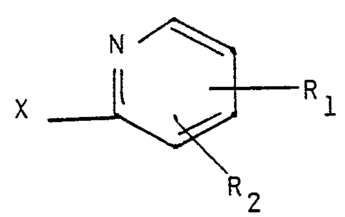

(V)

worin X für Halogen steht und $R_1$ bis $R_4$ die obige Bedeutung haben, durch Zusammenschmelzen oder Erhitzen in einem geeigneten Lösungsmittel, z. B. in verdünnter alkoholischer Lösung, in Gegenwart einer Säure, direkt in die verbindungen der Formel I überführen. Das Zusammenschmelzen geschieht bei Temperaturen zwischen ca. 120 und 200 °C, vorzugsweise bei 160-175 °C. Die Umsetzung in Lösung erfolgt zweckmäßig bei der Siedetemperatur unter Säurezusatz.

Die Ausgangsstoffe für die erfindungsgemäßen Verfahren können — soweit sie nicht bekannt sind — , nach üblichen Verfahren erhalten werden.

Für die Synthese der Verbindungen IIa/IIb kann man entsprechend dem folgenden Muster vorgehen :

(Siehe Schema Seite 5 f.)

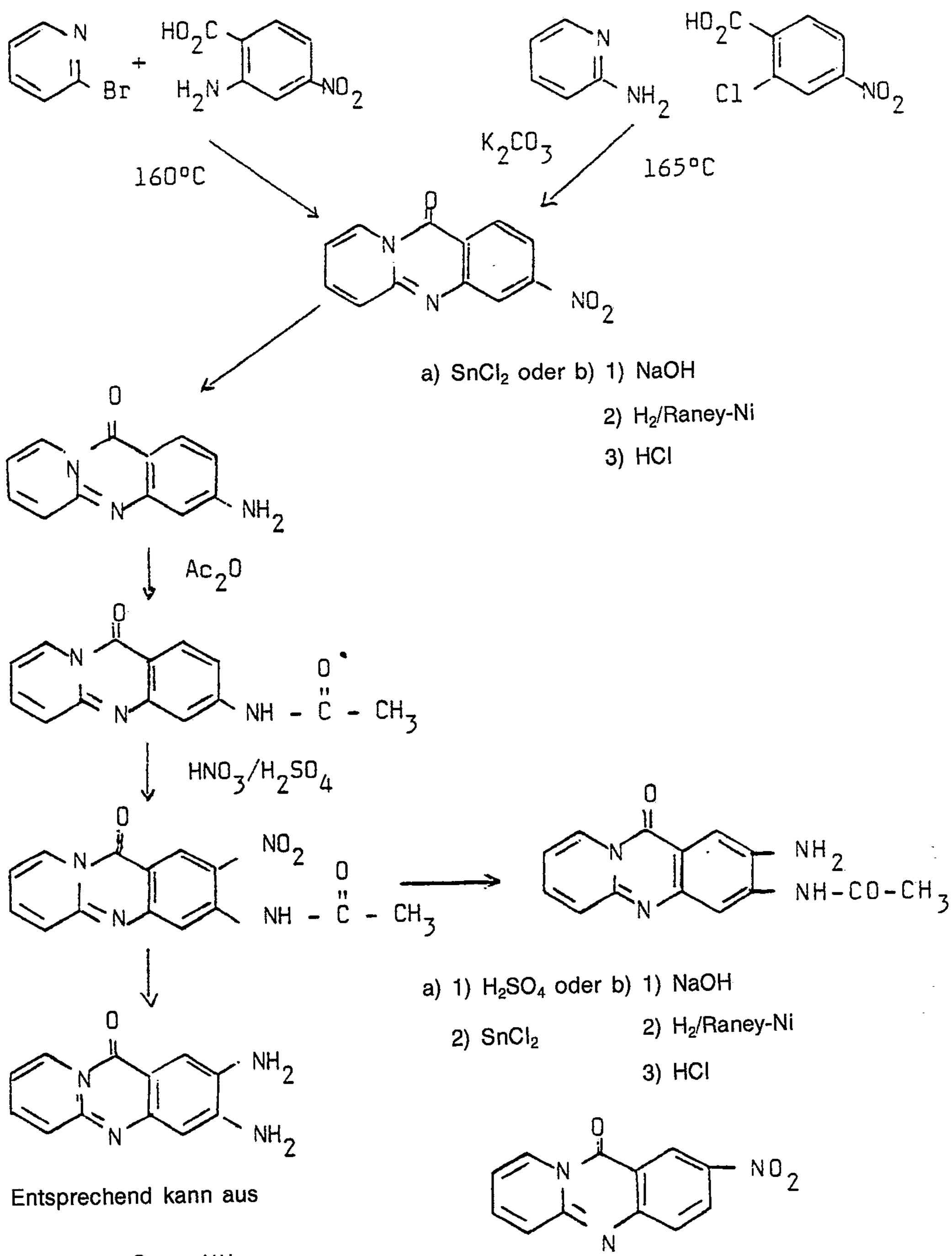


Entsprechend kann aus

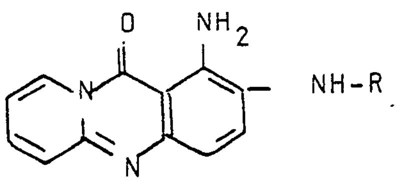

(R = H oder Acyl, z. B. Acetyl) erhalten werden.

Die Triazolverbindungen, die bei Verfahren 2 als Vorstufen für Verbindung III dienen, können beispielsweise folgendermaßen aufgebaut werden :

(Siehe Schema Seite 6 f.)

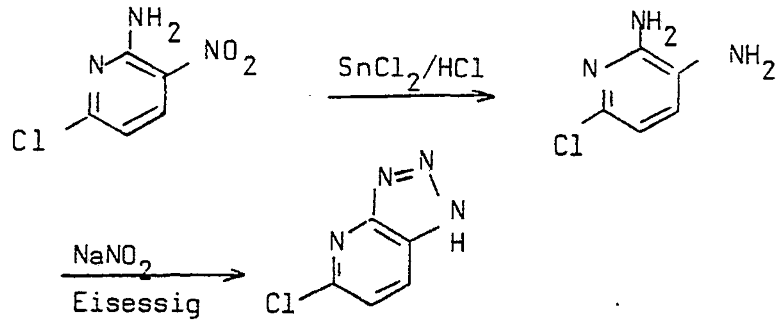


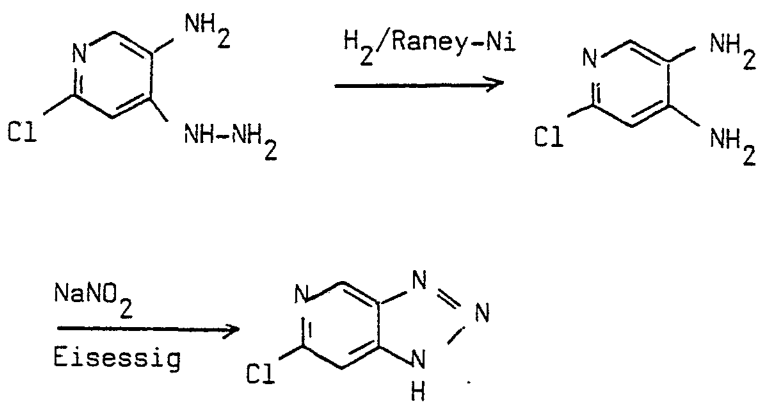


Die erfindungsgemäßen Verbindungen sind vor allem therapeutisch verwendbar, insbesondere als antiallergische Mittel. Wie der Test auf die passive cutane Anaphylaxie (PCA-Test) an der Ratte zeigt, sind die Verbindungen — im Gegensatz zu dem Handelsprodukt Cromoglycinsäure — auch oral wirksam und haben eine lange Wirkungsdauer. Die $ED_{50}$ (oral) an der Ratte wurde beispielsweise mit 0,29 mg/kg für die Verbindung nach Beispiel 4 und mit 0,25 mg/kg für die Verbindung nach Beispiel 4a ermittelt.

Die Verbindungen der Formel I sind daher wertvolle Arzneistoffe. Von besonderem Wert sind sie bei der Prophylaxe und Behandlung allergischer Krankheiten wie Asthma oder auch Heufieber, Conjunctivitis, Urticaria, Ekzemen, atopischer Dermatitis. Sie können auch muskelrelaxierend (bronchodilatorisch) und vasodilatorisch wirken.

Die prophylaktische oder therapeutische Dosis pro Kilogramm ist abhängig von der Substanz, von der Beschaffenheit und Ernsthaftigkeit des allergischen Zustands und von der Verabreichungsart. Die Einzeldosis beträgt, unter Berücksichtigung der vorstehenden Bemerkungen, pulmonal 0,25-50 mg, oral 5-200 mg. Nasal und okular werden die erfindungsgemäßen Verbindungen in gepufferter wäßriger Lösung mit 0,5 bis 5 % Wirkstoff angewendet.

Die für die verschiedenen Anwendungsweisen geeigneten galenischen Zubereitungen enthalten neben den Verbindungen der Formel I übliche Hilfs- und/oder Trägerstoffe und gegebenenfalls weitere Arzneistoffe. Möglich sind u.a. Kombinationen mit $\beta_2$-Adrenergika, Xanthinen, Anticholinergika.

Für die orale Verabreichung eignen sich Kapseln, Tabletten, Lösungen oder Suspensionen.

Bei der pulmonalen Gabe werden vorzugsweise trockene Pulver mit einem Teilchendurchmesser von 0,5-7 μm mittels Treibgasen oder unter Verwendung von Pulverinhalatoren in den Bronchialbereich gebracht.

Für die lokale Anwendung werden Lotionen, Cremes, Salben, Emulsionen und Sprays benutzt.

Formulierungsbeispiele :

1. Tabletten

Zusammensetzung :

| | |
|---|---|
| Verbindungen nach Anspruch 1 | 0,10 g |
| Stearinsäure | 0,01 g |
| Glucose | 0,89 g |
| | 1,00 g |

Die Bestandteile werden in dem entsprechenden Mengenverhältnis in üblicher Weise zu Tabletten von 1,00 g verarbeitet. Der Wirkstoffanteil wird gewünschtenfalls auf Kosten des Glucoseanteils erhöht.

2. Inhalationsaerosol

Zusammensetzung

| | |
|---|---|
| Verbindung nach Anspruch 1 | 1,00 Gew.-Teile |
| Sojalecithin | 0,20 Gew.-Teile |
| Verflüssigte Treibgasmischung (Frigene 11, 12 und 114) | ad 100,00 Gew.-Teile |

Die Zubereitung wird in Aerosolbehälter mit Dosierventil abgefüllt. Das Ventil ist so ausgelegt, daß die Einzeldosis 5 mg Wirkstoff enthält.

Von den Verbindungen der Formel I sind besonders diejenigen von Interesse, die den Formeln Ia, Ib, Id und Ie, insbesondere Ia und Id entsprechen. In den Substituentenpaaren $R_1/R_2$ bzw. $R_3/R_4$ bedeutet der eine Rest bevorzugt Wasserstoff, während der andere H, Niederalkyl oder Niederalkoxy darstellt. Ist $R_1$ ein ankondensierter Benzolring, ist dieser bevorzugt in der 6/7- oder 2/3-Position der Formel I. Die niederen Alkyl- und niederen Alkoxyreste enthalten 1 bis 4 C-Atome. Halogen bedeutet Fluor, Chlor, Brom und Jod, bevorzugt Fluor und Chlor, insbesondere Chlor. Hervorzuheben sind H, Methoxy, Methyl und Isopropyl, wobei diese Reste bevorzugt in der 2- oder 8-Position stehen, während die Gruppe —N=N—NH— sich in der 1/2- oder 8/9-Position des Grundgerüsts befindet.

Für die erfindungsgemäßen Verbindungen wurde jeweils nur eine Formel angegeben. Es versteht sich jedoch, daß aufgrund der Triazolgruppierung jeweils zwei tautomere (prototrope) Formen existieren, beispielsweise

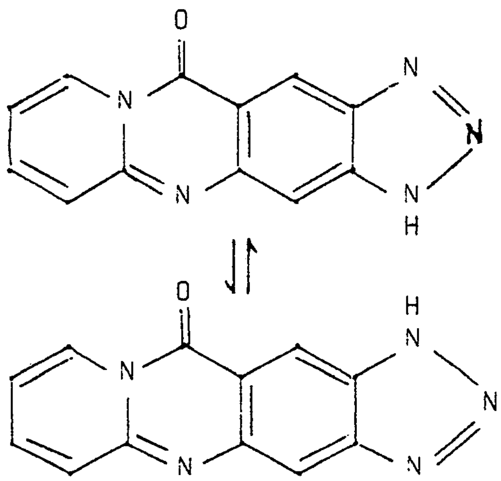

Die erfincungsgemäßen Verfahren werden in den nachstehenden Beispielen näher erläutert :

Beispiel 1

5-Oxo-5H-pyrido [2,1-b]-1H-triazolo [4,5-g] chinazolin

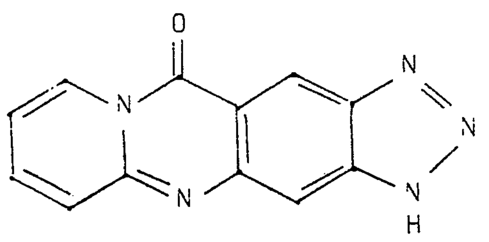

× 1/2 $CH_3OH$

5,98 g 11-Oxo-11H-2,3-diamino-pyrido [2,1-b] chinazolindihydrochlorid, 1,68 g Natriumacetat und 3,96 g Diphenylnitrosamin werden in 100 ml Eisessig eine Stunde auf 75-85 °C erhitzt. Danach wird auf 20 °C abgekühlt, der Niederschlag wird abgesaugt und gut mit Wasser gewaschen.

Die Rohausbeute von 4 g wurde mit 17 ml 1N-Natronlauge ins Natriumsalz überführt und über eine Kieselgelsäule mit Chloroform : Methanol = 4 : 1 gereinigt. Von der Kieselgelsäule wurden 1,5 g der Titelverbindung erhalten (Zersetzungspunkt 334-335 °C), die mit 1/2 Mol Methanol kristallisiert.

Analyse : $C_{12}H_7N_5O \times 1/2\ CH_3OH$

ber. : C 59,29 H 3,56 N 27,67 %

gef. : C 59,06 H 4,27 N 27,27 %

Beispiel 2

8-Methyl-5-oxo-5H-pyrido [2,1-b] 1H-triazolo [4,5-g] chinazolin

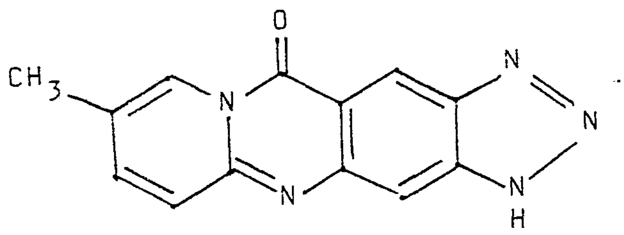

2,8 g 11-Oxo-11H-2,3-diamino-8-methyl-pyrido [2,1-b] chinazolindihydrochlorid werden in 18 ml Wasser und 7 ml Eisessig suspendiert und auf dem Wasserbad bis 40 °C erwärmt. Dann gibt man 0,7 g Natriumnitrit hinzu und rührt noch 60 Minuten. Die braunen Kristalle werden abgesaugt, mit Wasser gewaschen und im Vakuum bei 80 °C getrocknet.

Das Rohprodukt (1,9 g) wird über eine Kieselgelsäule mit Chloroform : Methanol : 25 %igem Ammoniak = 5 : 4 : 1 gereinigt.

Man erhält das Ammoniumsalz, das mit Eisessig in die Titelverbindung überführt wird.

Analyse : $C_{13}H_9N_5O$
ber. : C 62,15 H 3,59 N 27,89 %
gef. : C 62,21 H 3,49 N 28,12 %

Beispiel 3

4-Oxo-4H-pyrido [2,1-b] 1H-triazolo [4,5-f] chinazolin

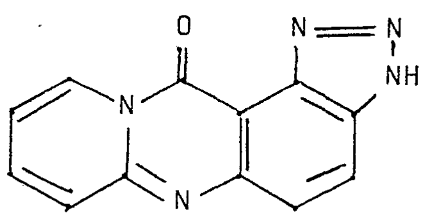

1,7 g 11-Oxo-11H-1-amino-2-acetamido-pyrido [2,1-b] chinazolin werden in einem Gemisch von 10 ml Eisessig und 15 ml Wasser gelöst und auf dem Wasserbad auf 60 °C erhitzt. Dann wird 1,0 g Natriumnitrit hinzugegeben, 1 Stunde gerührt und, wie im Beispiel 2 angegeben, aufgearbeitet. Fp. ≫ 380 °C (Zers.).

Analyse : $C_{12}H_7N_5O \times 1/2\ H_2O$
ber. : C 58,54 H 3,25 N 28,46 %
gef. : C 58,76 H 3,25 N 28,57 %

Analog erhält man :

Beispiel 3a)

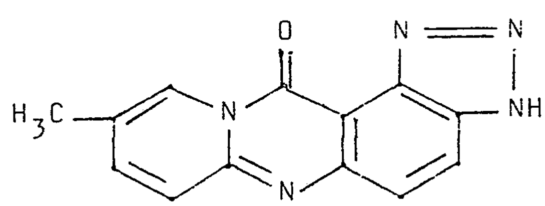

Fp. > 350 °C (Zers.)

Analyse : $C_{13}H_9N_5O$
ber. : C 62,15 H 3,59 N 27,89 %
gef. : C 62,28 H 3,59 N 27,29 %

Entsprechend den vorstehenden Beispielen erhält man auch die nachstehenden Verbindungen

(Siehe Schema Seite 9 f.)

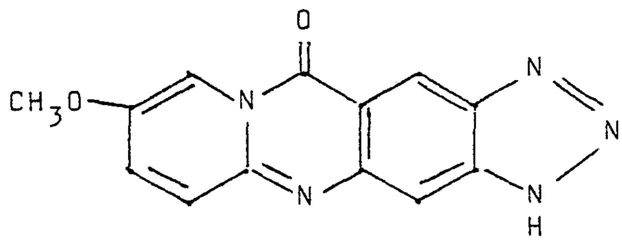

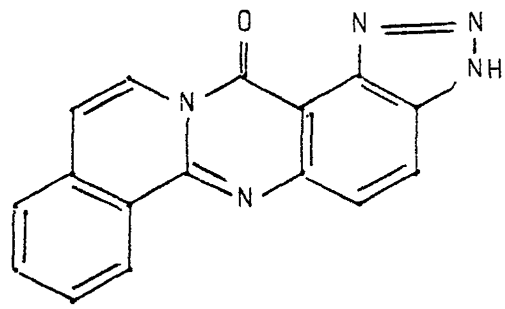

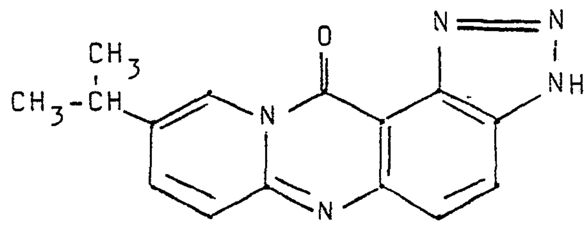

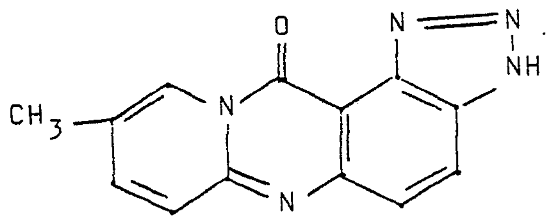

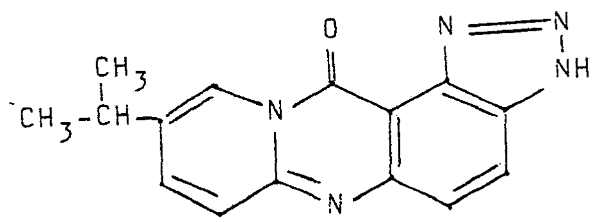

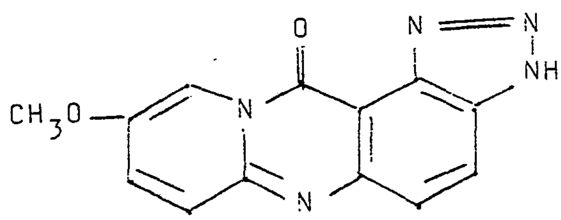

Beispiel 4

5-Oxo-5H-triazolo [5,4-e] pyrido [2,1-b] chinazolin

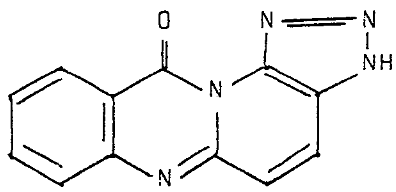

5,74 g 5-Chlor-1H-triazolo [4,5-b] pyridin und 5,48 g Anthranilsäure werden in 120 ml Ethanol und 12 ml 32 %iger Salzsäure 50 Stunden am Rückfluß gekocht. Die ausgefallenen Kristalle des Hydrochlorids werden abgesaugt und mit Natriumacetat in die Titelverbindung überführt. Man erhält 2 g der gewünschten Verbindung, die mit 1 Mol Kristallwasser kristallisiert. Zersetzungspunkt 320 °C.

Analyse : $C_{12}H_7N_5O \times 1\ H_2O$
ber. : C 56,47 H 3,53 N 27,45 %
gef. : C 59,14/59,48 H 3,53/3,35 N 27,98 %

In gleicher Weise wird hergestellt :

a) 7-Isopropyl-5-oxo-5H-triazolo [5,4-e] pyrido [2,1-b]-chinazolin, Zersetzungspunkt 281-286 °C ;

Analyse : $C_{15}H_{13}N_5O$
ber. : C 64,52 H 4,66 N 25,09 %
gef. : C 64,15 H 4,66 N 25,20 %

b) 7-Methyl-5-oxo-5H-triazolo [5,4-e] pyrido [2,1-b]-chinazolin-hydrochlorid ; Zersetzungspunkt 355 °C ;

Analyse : $C_{13}H_9N_5O \times HCl$
ber. : C 54,26 H 3,48 N 24,35 Cl 12,35 %
gef. : C 53,74 H 3,38 N 24,11 Cl 12,33 %

Ferner die Verbindungen der nachstehenden Formeln :

c) 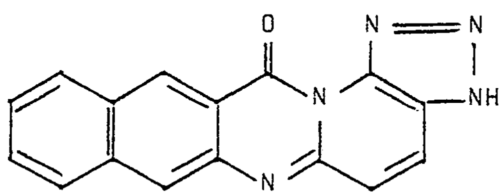 × HCl × $H_2O$

Fp. 380 °C (Zers.)

Analyse : $C_{16}H_9N_5O \times HCl \times H_2O$
ber. : C 56,22 H 3,51 N 20,50 Cl 10,50 %
gef. : C 56,51 H 3,27 N 19,90 Cl 10,16 %

d) 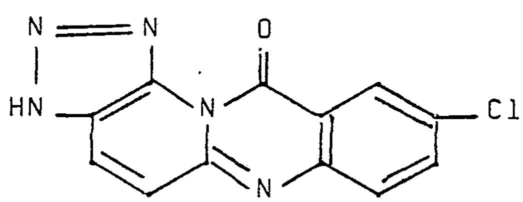 × HCl

Fp. 380 °C (Zers.)

Analyse : $C_{12}H_6ClN_5O \times HCl$
ber. : C 46,75 H 2,27 N 22,73 Cl 23,05 %
gef. : C 47,19 H 2,32 N 22,66 Cl 21,11 %

(Siehe Schema Seite 11 f.)

e) 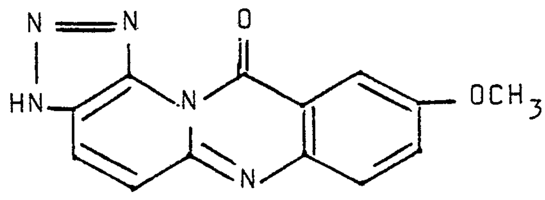

Fp. 380 °C (Zers.)

Analyse : $C_{13}H_9N_5O_2$
ber. : C 58,43 H 3,37 N 26,22 %
gef. : C 58,28 H 3,27 N 26,48 %

f) 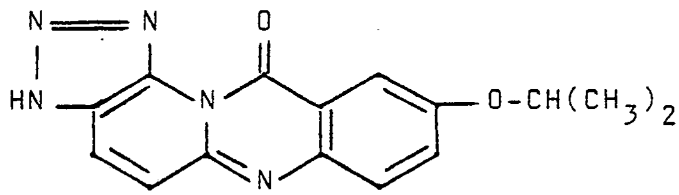

Fp. 380 °C (Zers.)

Analyse : $C_{15}H_{13}N_5O \times HCl$
ber. : C 54,30 H 4,22 N 21,12 Cl 9,65 %
gef. : C 54,90 H 4,31 N 21,52 Cl 9,76 %

**Patentansprüche**

1. Verbindungen der Formel

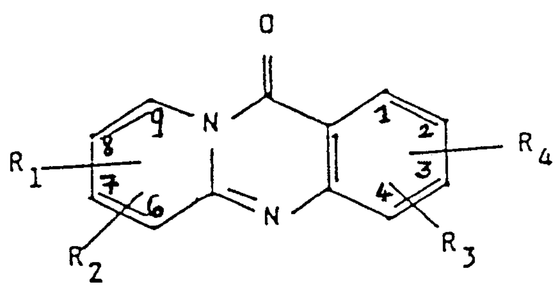

(I)

in der entweder $R_1$ und $R_2$ für Wasserstoff, $C_1$-$C_4$-Alkyl, $C_1$-$C_4$-Alkoxy, $R_1$ auch für Halogen in 8-Position (nach der Numerierung in Formel I), $R_2$ auch für einen ankondensierten Benzolring und $R_3$ und $R_4$ gemeinsam für die Gruppe —N=N—NH— stehen, oder $R_1$ und $R_2$ gemeinsam die Gruppe —N=N—NH— bedeuten und $R_3$ und $R_4$ Wasserstoff, $C_1$-$C_4$-Alkyl, $C_1$-$C_4$-Alkoxy, $R_3$ auch Halogen in 2-, 3- oder 4-Position (nach der Numerierung in Formel I), $R_4$ auch einen ankondensierten Benzolring darstellen, sowie die Salze dieser Verbindungen mit basischen Stoffen.

2. Arzneimittel, gekennzeichnet durch einen Gehalt an einer Verbindung nach Anspruch 1 neben üblichen Hilfs- und/oder Trägerstoffen.

3. Arzneimittel nach Anspruch 2, dadurch gekennzeichnet, daß es zusätzlich einen Wirkstoff aus der Gruppe der $\beta_2$-Adrenergika, der Xanthine oder der Anticholinergika enthält.

4. Verbindungen nach Anspruch 1 zur Anwendung bei der Behandlung allergischer Zustände.

5. Verfahren zur Herstellung von Verbindungen nach Anspruch 1, in der entweder $R_1$ und $R_2$ für Wasserstoff, $C_1$-$C_4$-Alkyl, $C_1$-$C_4$-Alkoxy, $R_1$ auch für Halogen in 8-Position (nach der Numerierung in Formel I), $R_2$ auch für einen ankondensierten Benzolring und $R_3$ und $R_4$ gemeinsam für die Gruppe —N=N—NH— stehen oder $R_1$ und $R_2$ gemeinsam die Gruppe —N=N—NH— bedeuten und $R_3$ und $R_4$ Wasserstoff, $C_1$-$C_4$-Alkyl, $C_1$-$C_4$-Alkoxy, $R_3$ auch Halogen in 2-, 3- oder 4-Position (nach der Numerierung in Formel I), $R_4$ auch einen ankondensierten Benzolring darstellen, sowie die Salze dieser Verbindungen mit basischen Stoffen, dadurch gekennzeichnet, daß man

a) eine Diaminoverbindung der Formel

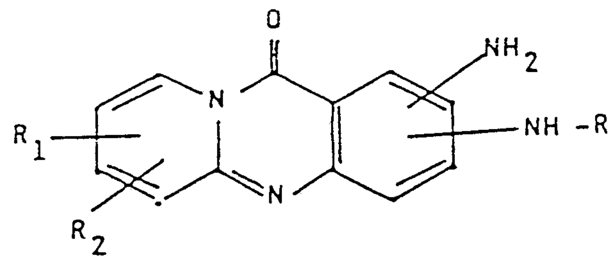

(IIa)

bzw.

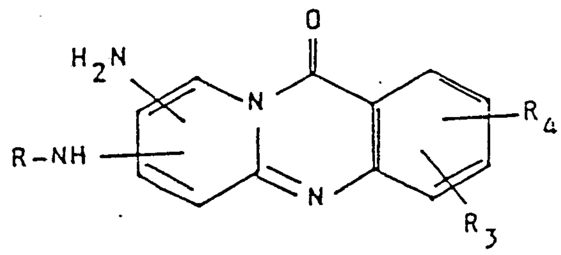

(R = H oder Acyl)

(IIb)

worin $R_1$ und $R_2$ bzw. $R_3$ und $R_4$ die obige Bedeutung haben und die Aminogruppen sich jeweils an benachbarten C-Atomen befinden, in Gegenwart von Säure bei Temperaturen zwischen etwa 0 und 100 °C mit nitrosierenden Agenzien umsetzt oder daß man

b) eine Verbindung der Formel

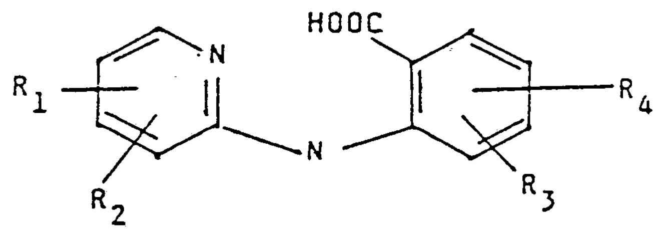

(III)

worin $R_1$ bis $R_4$ die obige Bedeutung haben, durch Einwirkung von Säure cyclisiert.

6. 5-Oxo-5H-pyrido [2,1-b]-1H-triazolo [4,5-g] chinazolin.
7. 8-Methyl-5-oxo-5H-pyrido [2,1-b]-1H-triazolo [4,5-g] chinazolin.
8. 4-Oxo-4H-pyrido [2,1-b] 1H-triazolo [4,5-f] chinazolin.
9. 5-Oxo-5H-triazolo [5,4-e] pyrido [2,1-b] chinazolin.
10. 7-Isopropyl-5-oxo-5H-triazolo [5,4-e] pyrido [2,1-b] chinazolin.

**Claims**

1. Compounds of formula

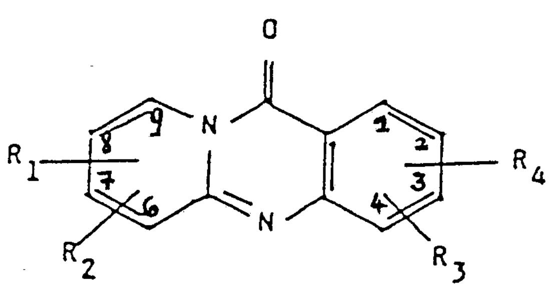

(I)

wherein either $R_1$ and $R_2$ represent hydrogen, $C_{1-4}$ alkyl, $C_{1-4}$ alkoxy and $R_1$ also represents halogen in the 8 position (according to the numbering in formula I), $R_2$ also represents a fused benzene ring and $R_3$ and $R_4$ together represent the group —N=N—NH—, or $R_1$ and $R_2$ together represent the group —N=N—NH— and $R_3$ and $R_4$ represent hydrogen, $C_{1-4}$ alkyl, $C_{1-4}$ alkoxy, $R_3$ also represents halogen in

the 2, 3 or 4 position (according to the numbering in formula I), $R_4$ also represents a fused benzene ring, and the salts of these compounds with basic substances.

2. Pharmaceutical compositions characterised in that they contain a compound as claimed in claim 1 together with conventional excipients and/or carriers.

3. Pharmaceutical composition as claimed in claim 2, characterised in that it additionally contains an active substance selected from the group comprising the $\beta_2$-adrenergics, the xanthines or the anticholinergics.

4. Compounds as claimed in claim 1 for use in the treatment of allergic conditions.

5. Process for preparing compounds as claimed in claim 1 wherein either $R_1$ and $R_2$ represent hydrogen, $C_{1-4}$ alkyl, $C_{1-4}$ alkoxy and $R_1$ also represents halogen in the 8 position (according to the numbering in formula I), $R_2$ also represents a fused benzene ring and $R_3$ and $R_4$ together represent the group —N=N—NH—, or $R_1$ and $R_2$ together represent the group —N=N—NH— and $R_3$ and $R_4$ represent hydrogen, $C_{1-4}$ alkyl, $C_{1-4}$ alkoxy, $R_3$ also represents halogen in the 2, 3 or 4 position (according to the numbering in formula I), $R_4$ also represents a fused benzene ring, and the salts of these compounds with basic substances, characterised in that

a) a diamino compound of formula

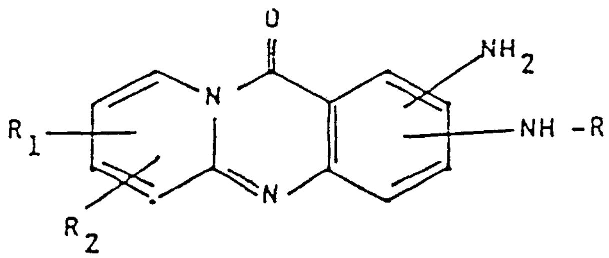

(IIa)

or

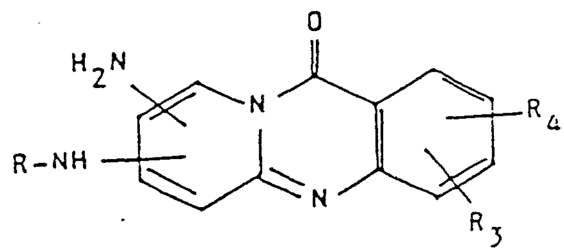

(R = H or acyl)

(IIb)

wherein $R_1$ and $R_2$ or $R_3$ and $R_4$ are as hereinbefore defined and the amino groups are located at adjacent carbon atoms, is reacted in the presence of acid at temperatures of between about 0 and 100 °C with nitrosating agents or

b) a compound of formula

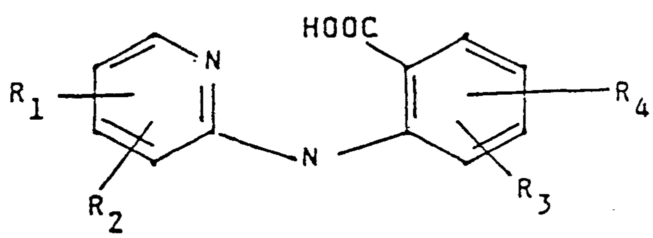

(III)

wherein $R_1$ to $R_4$ are as hereinbefore defined, is cyclised by the action of acid.

6. 5-Oxo-5H-pyrido [2,1-b]-1H-triazolo [4,5-g] quinazoline.

7. 8-Methyl-5-oxo-5H-pyrido [2,1-b]-1H-triazolo [4,5-g]-quinazoline.

8. 4-Oxo-4H-pyrido [2,1-b]-1H-triazolo [4,5-f] quinazoline.

9. 5-Oxo-5H-triazolo [5,4-e] pyrido [2,1-b] quinazoline.

10. 7-Isopropyl-5-oxo-5H-triazolo [5,4-e] pyrido [2,1-b]-quinazoline.

**Revendications**

1. Composés de formule

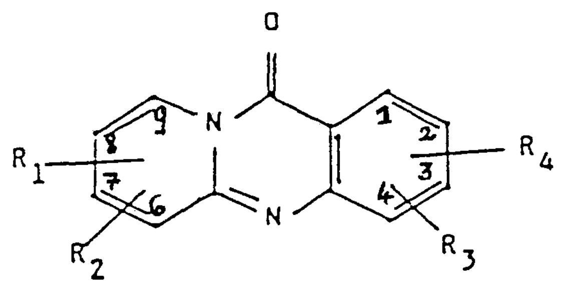

(I)

dans laquelle soit $R_1$ et $R_2$ remplacent hydrogène, alcoyle en $C_1$-$C_4$, alcoxy en $C_1$-$C_4$, $R_1$ remplace également halogène en position 8 (selon la numérotation dans la formule I), $R_2$ remplace également un cycle benzène condensé et $R_3$ et $R_4$ remplacent ensemble le groupe —N=N—NH—, soit $R_1$ et $R_2$ représentent ensemble le groupe —N=N—NH— et $R_3$ et $R_4$ représentent hydrogène, alcoyle en $C_1$-$C_4$, alcoxy en $C_1$-$C_4$, $R_3$ représente également halogène en position 2, 3 ou 4 (selon la numérotation dans la formule I), $R_4$ représente également un cycle benzène condensé, ainsi que les sels de ces composés avec des substances basiques.

2. Médicament caractérisé par une teneur en un composé selon la revendication 1, conjointement à des adjuvants et/ou excipients usuels.

3. Médicament selon la revendication 2, caractérisé en ce qu'il contient en outre une substance active du groupe des $\beta_2$-adrénergiques, des xanthines ou des anticholinergiques.

4. Composés selon la revendication 1 pour l'utilisation dans le traitement des états allergiques.

5. Procédé pour la préparation de composés selon la revendication 1, dans laquelle soit $R_1$ et $R_2$ remplacent hydrogène, alcoyle en $C_1$-$C_4$, alcoxy en $C_1$-$C_4$, $R_1$ remplace également halogène en position 8 (selon la numérotation dans la formule I), $R_2$ remplace également un cycle benzène condensé et $R_3$ et $R_4$ remplacent ensemble le groupe —N=N—NH—, soit $R_1$ et $R_2$ représentent ensemble le groupe —N=N—NH—, et $R_3$ et $R_4$ représentent hydrogène, alcoyle en $C_1$-$C_4$, alcoxy en $C_1$-$C_4$, $R_3$ représente également halogène en position 2, 3 ou 4 (selon la numérotation dans la formule I), $R_4$ représente également un cycle benzène condensé, ainsi que les sels de ces composés avec des substances basiques, caractérisé en ce que

a) on fait réagir un composé diamino de formule

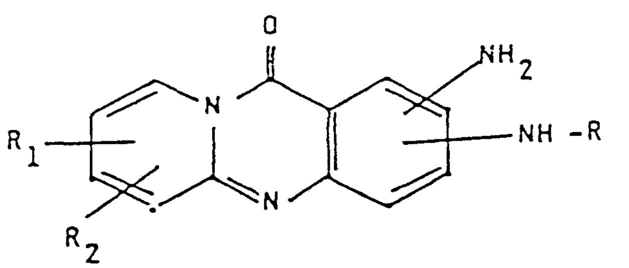

(IIa)

ou

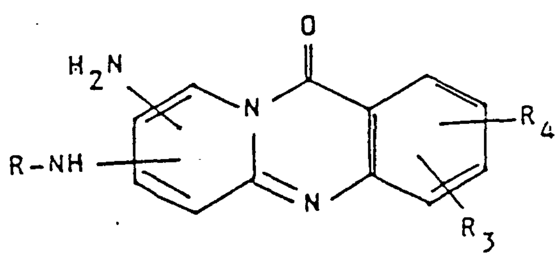

(R = H ou acyle)

(IIb)

où $R_1$ et $R_2$ ou respectivement $R_3$ et $R_4$ ont la signification ci-dessus et les groupes amino se trouvent dans chaque cas sur des atomes de C voisins, en présence d'acide à des températures entre environ 0 et 100 °C, avec des agents nitrosants ou en ce que

b) on cyclise un composé de formule

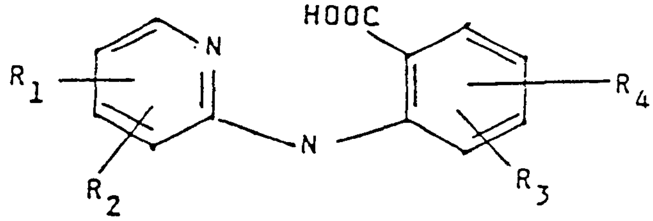

(III)

dans laquelle $R_1$ à $R_4$ ont la signification ci-dessus, par action d'un acide.

6. La 5-oxo-5H-pyrido [2,1-b]-1H-triazolo [4,5-g] quinazoline.
7. La 8-méthyl-5-oxo-5H-pyrido [2,1-b]-1H-triazolo [4,5-g] quinazoline.
8. La 4-oxo-4H-pyrido [2,1-b] 1H-triazolo [4,5-f] quinazoline.
9. La 5-oxo-5H-triazolo [5,4-e] pyrido [2,1-b] quinazoline.
10. La 7-isopropyl-5-oxo-5H-triazolo [5,4-e] pyrido [2,1-b] quinazoline.